# EUROPEAN PATENT SPECIFICATION

(11) **EP 2 451 531 B1**
(45) Date of publication and mention of the grant of the patent: **30.03.2016**
(21) Application number: 10723061.7
(22) Date of filing: 20.05.2010
(51) Int. Cl.: A61Q 5/06, A61K 8/49, A61K 8/60

(54) **METHOD OF TREATING HAIR**
HAARBEHANDLUNGSVERFAHREN
PROCÉDÉ DE TRAITEMENT DES CHEVEUX

(30) Priority: 09.07.2009 EP 09165023
(43) Date of publication of application: 16.05.2012
(73) Proprietor: Unilever PLC, London EC4Y 0DY (GB); Unilever N.V., 3013 AL Rotterdam (NL)
(72) Inventor: PAUL, Prem, Kumar, Cheyalazhagan, Merseyside CH63 3JW (GB)
(74) Representative: James, Helen Sarah
(86) International application number: PCT/EP2010/056954
(87) International publication number: WO 2011/003665

(56) References cited:
- EP-A- 1 604 659
- EP-A- 1 806 121
- WO-A-2008/110399

## Description

The invention relates to a method of styling hair. The invention is particularly advantageous in relation to hair straightening.

The styling market can be classified in various sub-sets based on the desired styling effect; one such sub-set is products for straightening the hair.

A problem with straightened hair is that once the straightening process has taken place it tends to increase in volume and appear fluffy, this is especially troublesome in humid conditions.

Sugars and sugar derivatives are one class of the countless number of compounds that have been added to hair care compositions.

WO2004/037217 describes heat activated durable styling compositions comprising saccharides and film forming agents.

WO2008/110399 describes straightening compositions comprising lactulose. However there is a need to enhance the straightening effect achieved by such a system.

The present invention relates to hair straightening compositions giving improved humidity resistance to the straightened hair thus retaining its style.

The present invention relates to a method of styling hair comprising the following steps:
i) using a crystalline organic material or mixtures thereof to manufacture a hair care composition; wherein the crystalline organic material or mixtures thereof comprises pipecolic acid and has an intrinsic property of taking up water at 25°C and 70% relative humidity of at least 10 wt% of its total weight at 0.1% relative hymidity, and
ii) applying said hair composition to the hair;

The invention further relates to the use of the crystalline organic material described above for straightening hair and imparting humidity resistance to hair.

The invention relates to a crystalline organic material or mixtures thereof having a total water uptake at 25°C and 70% relative humidity of at least 10 wt% of its total weight at 0.1% relative humidity, preferably the crystalline organic material or mixtures thereof have a total water uptake at 25°C and 90% relative humidity of at least 10 wt% of its total weight at 0.1 % relative humidity. The crystalline organic material or mixture thereof comprises pipecolic acid (piperidine 2-carboxylic acid). It should that be noted that the crystalline organic material is usually in solution within the hair care composition, however when applied the hair and the solvent is removed (during the hair drying stage) it could re-crystallise. If excessive heat is used it is thought that the material could transform into a glassy state on the hair.

Preferably the crystalline organic material has a melting point above 40°C, more preferably above 50°C.

Mixtures of the crystalline organic materials descried above are advantageous as they exhibit synergistic results regarding styling benefits. Mixtures where at least one ingredient is a sugar are particularly advantageous, especially if the sugar is a di or trisaccharide.

Synergistic effects relating to a decrease in volume can be seen when pipecolic acid is combined with a sugar. Particularly preferred sugars are selected from the group consisting of isomalt and its constituent sugars glucose mannitol or glucose sorbitol, trehalose, raffinose, lactulose or mixtures thereof, lactulose or raffinose being particularly preferred. Lactulose is especially effective.

It is preferable if the ratio of pipecolic acid to sugar is from 1:4 to 4:1, more preferably from 2:1 to 1:2, most preferably from 3:3 to 2:3.

The level of organic crystalline material in the total composition is preferably from 0.001 to 10 wt%, more preferably from 0.1 to 5 wt%, most preferably from 0.2 wt% to 3 wt%.

The formulation may include conditioning materials such as surfactants, cationic conditioners suitable for hair, quaternary silicone polymers, silicone based conditioners and their emulsions, and amino functional silicones and their emulsions.

The formulation may include conditioning materials such as surfactants, cationic conditioners suitable for hair, quaternary silicone polymers, silicone based conditioners and their emulsions, and amino functional silicones and their emulsions. Silicone based products are particularly preferred.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

The viscosity of the emulsified silicone itself (not the emulsion or the final hair conditioning composition) is typically at least 10,000 cst. The viscosity of the silicone itself is preferably at least 60,000 cst, most preferably at least 500,000 cst, ideally at least 1,000,000 cst. Preferably the viscosity does not exceed 10⁹ cst for ease of formulation.

Emulsified silicones for use the invention will typically have an average silicone particle size in the composition of less than 30, preferably less than 20, more preferably less than 10 microns. Most preferably the average silicone particle size of the emulsified silicone in the composition is less than 2 microns, ideally it ranges from 0.01 to 1 micron. Silicone emulsions having an average silicone particle size of ≤ 0.15 microns are generally termed microemulsions.

Particle size may be measured by means of a laser light scattering technique, using a 2600D Particle Sizer from Malvern Instruments.

Suitable silicone emulsions for use in the invention are also commercially available in a pre-emulsified form.

Examples of suitable pre-formed emulsions include emulsions DC2-1766, DC2-1784, and microemulsions DC2-1865 and DC2-1870, all available from Dow Corning. These are all emulsions/microemulsions of dimethiconol. Cross-linked silicone gums are also available in a pre-emulsified form, which is advantageous for ease of formulation. A preferred example is the material available from Dow Corning as DC X2-1787, which is an emulsion of cross-linked dimethiconol gum. A further preferred example is the material available from Dow Corning as DC X2-1391, which is a microemulsion of cross-linked dimethiconol gum.

A further preferred class of silicones for inclusion in the invention are amino functional silicones. By "amino functional silicone" is meant a silicone containing at least one primary, secondary or tertiary amine group, or a quaternary ammonium group.

Examples of suitable amino functional silicones include:
(i) polysiloxanes having the CTFA designation "amodimethicone", and the general formula:

   HO-[Si(CH₃)₂-O-]ₓ-[Si(OH)(CH₂CH₂CH₂-NH-CH₂CH₂NH₂)-O-]_{y}-H

   in which x and y are numbers depending on the molecular weight of the polymer, generally such that the molecular weight is between about 5,000 and 500,000.
(ii) polysiloxanes having the general formula:

   R'ₐG₃₋ₐ-Si(OSiG₂)ₙ-(OSiGbR'_{2-b})ₘ-O-SiG₃₋ₐ-R'ₐ

   in which:
   G is selected from H, phenyl, OH or C₁₋₈ alkyl, e.g. methyl;
   a is 0 or an integer from 1 to 3, preferably 0;
   b is 0 or 1, preferably 1;
   m and n are numbers such that (m + n) can range from 1 to 2000, preferably from 50 to 150;
   m is a number from 1 to 2000, preferably from 1 to 10;
   n is a number from 0 to 1999, preferably from 49 to 149, and
   R is a monovalent radical of formula -C_{q}H_{2q}L in which q is a number from 2 to 8 and L is an aminofunctional group selected from the following:
      -NR"-CH₂-CH₂-N(R")₂
      -N(R")₂
      -N"(R)₃A⁻
      -N⁺H(R")₂A⁻
      -N⁺H₂(R")A⁻
      -N(R")-CH₂-CH₂-N⁺H₂(R") A⁻
      in which R" is selected from H, phenyl, benzyl, or a saturated monovalent hydrocarbon radical, e.g. C₁₋₂₀ alkyl, and;
      A is a halide ion, e.g. chloride or bromide.

   Suitable amino functional silicones corresponding to the above formula include those polysiloxanes termed "trimethylsilylamodimethicone" as depicted below, and which are sufficiently water insoluble so as to be useful in compositions of the invention:

   Si(CH₃)₃-O-[Si(CH₃)₂-O-]ₓ-[Si(CH₃)(R-NH-CH₂CH₂NH₂)-O-]_{y}-Si (CH₃)₃

   wherein x + y is a number from about 50 to about 500, and wherein R is an alkylene group having from 2 to 5 carbon atoms. Preferably, the number x + y is in the range of from about 100 to about 300.
(iii) quaternary silicone polymers having the general formula:

   {(R¹)(R²)(R³)N⁺CH₂CH(OH)CH₂O(CH₂)₃[Si(R⁴)(R⁵)-O-]ₙ-Si(R⁶)(R⁷)-(CH₂)₃-O-CH₂CH(OH)CH₂N+(R⁸)(R⁹)(R¹⁰)} (X⁻)₂

   wherein R¹ and R¹⁰ may be the same or different and may be independently selected from H, saturated or unsaturated long or short chain alk(en)yl, branched chain alk(en)yl and C₅-C₈ cyclic ring systems;
   R² thru' R⁹ may be the same or different and may be independently selected from H, straight or branched chain lower alk(en)yl, and C₅-C₈ cyclic ring systems; n is a number within the range of about 60 to about 120, preferably about 80, and
   X- is preferably acetate, but may instead be for example halide, organic carboxylate, organic sulphonate or the like.

Suitable quaternary silicone polymers of this class are described in EP-A-0 530 974.

Amino functional silicones suitable for use in the invention will typically have a mole % amine functionality in the range of from about 0.1 to about 8.0 mole %, preferably from about 0.1 to about 5.0 mole %, most preferably from about 0.1 to about 2.0 mole %. In general the amine concentration should not exceed about 8.0 mole % since we have found that too high an amine concentration can be detrimental to total silicone deposition and therefore conditioning performance.

The viscosity of the amino functional silicone can suitably range from about 100 to about 500,000 cst.

Specific examples of amino functional silicones suitable for use in the invention are the aminosilicone oils DC2-8220, DC2-8166, DC2-8466, and DC2-8950-114, DC7134 (all ex Dow Corning), and GE 1149-75, (ex General Electric Silicones).

Also suitable are emulsions of amino functional silicone oils with non ionic and/or cationic surfactant.

Suitably such pre-formed emulsions will have an average amino functional silicone particle size in the shampoo composition of less than 30, preferably less than 20, more preferably less than 10 microns. Most preferably the average amino functional silicone particle size in the composition is less than 2 microns, ideally it ranges from 0.01 to 1 micron. Silicone emulsions having an average silicone particle size of ≤ 0.15 microns are generally termed microemulsions.

Pre-formed emulsions of amino functional silicone are also available from suppliers of silicone oils such as Dow Corning and General Electric. Specific examples include DC959 emulsion, DC929 Cationic Emulsion, DC939 Cationic Emulsion, and the non-ionic emulsions DC2-7224, DC2-8467, DC2-8177 and DC2-8154 (all ex Dow Coming).

The total amount of silicone incorporated into compositions of the invention depends on the level of conditioning desired and the material used. A preferred amount is from 0.01 to about 10% by weight of the total composition.

We have found that a total amount of silicone of from 0.3 to 5%, preferably 1 to 8%, by weight of the total composition is a suitable level.

The composition may comprises a styling aid. Particularly useful as styling aids with this invention are hair styling polymers. Hair styling polymers are well known articles of commerce and many such polymers are available commercially which contain moieties which render the polymers cationic, anionic, amphoteric or nonionic in nature. The polymers may be synthetic or naturally derived.

Compositions of the present invention are formulated into hair care compositions, especially products with hair styling claims. The compositions are preferably for use in styling human hair and, more preferably, they are packaged and labeled as such.

It is preferred if the products are left on hair after application and not immediately washed off (within 10 minutes of application, preferably the hair is not rinsed within 2 hours of application).

Preferred product forms are leave on formulations such as gels, mousses, sprays and aerosols.

Such styling products frequently include a carrier and further additional components. The carriers and additional components required to formulate such products vary with product type and can be routinely chosen by one skilled in the art. The following is a description of some of these carriers and additional components.

Hair care compositions of the present invention can comprise a carrier, or a mixture of such carriers, which are suitable for application to the hair. The carriers are present at from about 0.5% to about 99.5%, preferably from about 5.0% to about 99.5%, more preferably from about 10.0% to about 98.0%, of the composition. As used herein, the phrase "suitable for application to hair" means that the carrier does not damage or negatively affect the aesthetics of hair or cause irritation to the underlying skin.

Compositions according to the invention may comprise a buffer or pH adjuster. Preferred buffers or pH adjusters include weak acids and bases such glycine/sodium hydroxide, citric acid, lactic acid, succinic acid, acetic salt and salts thereof. Frequently a mixture of buffering system is used such as sodium citrate and citric acid.

Carriers suitable for use with hair care compositions of the present invention include, for example, those used in the formulation of hair sprays, mousses, tonics, waters, creams gels, shampoos, conditioners, and rinses. The choice of appropriate carrier will depend on the particular product to be formulated. The carriers used herein can include a wide range of components conventionally used in hair care compositions. The carriers can contain a solvent to dissolve or disperse the styling compound being used, with water, the C₁-C₆ alcohols, lower alkyl acetate and mixtures thereof being preferred. The carriers can also contain a wide variety of additional materials such as acetone, hydrocarbons (such as isobutane, hexane, decene), halogenated hydrocarbons (such as Freons) and volatile silicones such as cyclomethicone.

When the hair care composition is a hair spray, tonic, gel, or mousse the preferred solvents include water, ethanol, volatile silicone derivatives, and mixtures thereof. The solvents used in such mixtures may be miscible or immiscible with each other. Mousses and aerosol hair sprays can also utilise any of the conventional propellants to deliver the material as a foam (in the case of a mousse) or as a fine, uniform spray (in the case of an aerosol hair spray). Examples of suitable propellants include materials such as trichlorofluoromethane, dichlorodifluoromethane, difluoroethane, dimethylether, propane, n-butane or isobutane. A tonic or hair spray product having a low viscosity may also utilise an emulsifying agent. Examples of suitable emulsifying agents include nonionic, cationic, anionic surfactants, or mixtures thereof. If such an emulsifying agent is used, it is preferably present at a level of from about 0.01 % to about 7.5% by weight based on total weight of the composition. The level of propellant can be adjusted as desired but is generally from about 3% to about 30% by weight based on total weight for mousse compositions and from about 15% to about 50% by weight based on total weight for aerosol hair spray compositions.

Hair styling creams or gels also typically contain a structurant or thickener, typically in an amount of from 0.01% to 10% by weight.

Suitable spray containers are well known in the art and include conventional, non-aerosol pump sprays i.e., "atomisers", aerosol containers or cans having propellant, as described above, and also pump aerosol containers utilising compressed air as the propellant.

Further general ingredients suitable for all product forms include, sun-screening agents, anti-dandruff actives, carboxylic acid polymer thickeners for hair shampoo and conditioner compositions and emulsifiers for emulsifying the various carrier components of the compositions of the invention.

The compositions of the present invention may also contain adjuncts suitable for hair care. Generally such ingredients are included individually at a level of up to 2, preferably up to 1 wt% of the total composition. Suitable hair care adjuncts, include amino acids, sugars and ceramides.

The method of the invention comprises applying compositions of the invention preferably followed by a heating step.

It can be advantageous if the treated hair is heated to a temperature above 100°C, preferably above 150°C, more preferably above 180°C. the use of straightening irons is a way of achieving the aforementioned temperatures.

The following non-limiting Examples further illustrate the preferred embodiments of the invention. All percentages referred to in the examples and throughout this specification are by weight based on total weight unless otherwise indicated.

### Examples

Water uptake of the actives singly or in combination (equal amounts by weight of each) at 70% relative humidity and 90% relative humidity were measured using Dyniamic Vapour Sorption instrument by Surface Measurement Systems. (SMS Ltd.). The DVS system was comprised of a microbalance with a computer controlled water vapour generator. The sample (typically 20 - 60 milligrams) was suspended from the balance and exposed sequentially to a range of humidities in a flowing gas stream (200 cc/min of nitrogen) at a constant temperature (25°C). The corresponding change in mass was recorded together with the %RH and the temperature.

The results were as follows:

| Material | %wt water uptake (relative to 0.1 %RH)at 70% relative humidity | %wt water uptake (relative to 0.1%RH) at 90% relative humidity |
|---|---|---|
| Lactulose | 1.04 | 26.82 |
| Raffinose | 6.55 | 6.65 |
| Pipecolic acid | 42.85 | 43.02 |
| Raffinose and Pipecolic acid | 30.04 | 32.40 |
| Lactulose and Pipecolic acid | 28.40 | 68.22 |

2 grams of dark brown European curly switches (wavy # 6) were dosed with 0.5gm solutions containing lactulose and/or pipecolic acid and styled straight using a hair dryer (temperature 60°C -100°C). The styled switches were placed in high humidity for 1 hour (80%RH and 30°C). The final volumes are shown in the table for treatments from solution (only water) and from a silicone solution.

| **BLOW DRYING experiments** | | | |
|---|---|---|---|
| **Treatment** | **Final volume*** | **average hypothetical volume of sugar and pipecolic acid** | **effect of combination %synergy** |
| water (no treatment) | 11680 | | |
| water + 2wt%raffinose | 11713 | | |
| water + 2wt%lactulose | 10704 | | |
| water + 2wt%pipecolic acid | 9157 | | |
| water + 1wt%raffinose + 1wt%pipecolic acid | 9996 | 10855 | 9 |
| water + 1wt%lactulose + 1wt%pipecolic acid | 7697 | 9931 | 29 |
| | | | |
| treatment1 = 2wt%DC959 silicone solution (no sugar or amino acid) | 11161 | | |
| treatment1 + 2wt%pipecolic acid | 10372 | | |
| treatment1 + 2wt%raffinose | 10078 | | |
| treatment1 + 2wt%lactulose | 8457 | | |
| treatment1 + 1wt%raffinose + 1wt%pipecolic acid | 9658 | 10225 | 6 |
| treatment1 + 1wt%lactulose + 1wt%pipecolic acid | 8082 | 9415 | 16 |

| | | | |
|---|---|---|---|
| * **in mm^2 after 1 hour at 80%RH and 30°C** | | | |

From the table it is clear that pipecolic acid shows anti-humidity benefits when used alone, while the combination of pipecolic acid with sugars shows synergistic benefits.

In the above examples pipecolic acid used is DL-pipecolic acid (Sigma-Aldrich) and DC959 is an amino silicone emulsion from Dow Corning containing Dimethylsiloxane with aminoethylaminopropyl silsesquioxane, hydroxy terminated; polyethoxylated alcohols; hexadecyltrimethylammonium chloride & methanol.

## Claims

1. A method of styling hair comprising the following steps:
i) using a crystalline organic material or mixtures thereof to manufacture a hair care composition; wherein the crystalline organic material or mixtures thereof comprises pipecolic acid and has the intrinsic property of taking up water at 25°C and 70% relative humidity of at least 10 wt% of its total weight at 0.1% relative humidity.
ii) applying said hair composition to the hair.

2. A method according to any preceding claim in which the organic material I) further comprises a sugar.

3. A method according to any preceding claim in which the sugar is a disaccharide or a trisachharide.

4. A method according to claim 1 in which the crystalline organic material further comprises a sugar selected from the group consisting of isomalt, trehalose, raffinose, lactulose and mixtures thereof.

5. A method according to claim 4 in which the sugar is lactulose.

6. A method according to any preceding claim in which the level of crystalline organic material or mixtures thereof in the total composition is from 0.1 to 5 wt%.

7. A method according to any preceding claim in which the composition further comprises a silicone.

8. Use of the crystalline organic material described in claim 1 for straightening hair.

9. Use of the crystalline organic material described in claim 1 for imparting humidity resistance to hair.

## Patentansprüche

1. Verfahren zum Haar-Styling, umfassend folgende Schritte:
i) Herstellen eines Haarpflegemittels unter Verwendung eines kristallinen organischen Materials oder Mischungen davon, wobei das kristalline organische Material oder Mischungen davon Pipecolinsäure enthalten und die spezifische Eigenschaft besitzen, bei 25°C und 70% relativer Feuchtigkeit Wasser in einer Menge von mindestens 10 Gew.% des gesamten Gewichts bei 0.1% relativer Feuchtigkeit aufzunehmen,
ii) Auftragen dieser Zusammensetzung auf das Haar.

2. Verfahren gemäß irgendeinem vorhergehenden Anspruch, wobei das Material i) zusätzlich Zucker enthält.

3. Verfahren gemäß irgendeinem vorhergehenden Anspruch, wobei der Zucker ein Disaccharid oder ein Trisaccharid ist.

4. Verfahren gemäß Anspruch 1, wobei das kristalline organische Material zusätzlich einen Zucker ausgewählt aus der Gruppe, bestehend aus Isomalt, Trehalose, Raffinose, Laktulose und Mischungen davon, umfasst.

5. Verfahren gemäß Anspruch 4, wobei der Zucker Laktulose ist.

6. Verfahren gemäß irgendeinem vorhergehenden Anspruch, wobei die Menge an kristallinem organischen Material oder Mischungen davon 0.1 bis 5 Gew.%, bezogen auf die gesamte Zusammensetzung, beträgt.

7. Verfahren gemäß irgendeinem vorhergehenden Anspruch, wobei die Zusammensetzung zusätzlich ein Silikon enthält.

8. Verwendung des kristallinen organischen Materials gemäß Anspruch 1 zum Glätten von Haar.

9. Verwendung des kristallinen organischen Materials beschrieben in Anspruch 1, um dem Haar Widerstand gegen Feuchtigkeit zu vermitteln.

## Revendications

1. Méthode de coiffage de cheveux comprenant les étapes suivantes consistant à :
i) utiliser une substance organique cristalline ou des mélanges de plusieurs d'entre elles pour fabriquer une composition de soin capillaire ; la substance organique cristalline ou des mélanges de plusieurs d'entre elles comprenant de l'acide pipécolique et ayant la propriété intrinsèque d'absorber de l'eau à 25 °C et une humidité relative de 70 % à hauteur d'au moins 10 % en poids de son poids total à une humidité relative de 0,1 % ;
ii) appliquer ladite composition capillaire sur les cheveux.

2. Méthode selon la revendication précédente, dans laquelle la substance organique i) comprend en outre un sucre.

3. Méthode selon l'une quelconque des revendications précédentes, dans laquelle le sucre est un disaccharide ou un trisaccharide.

4. Méthode selon la revendication 1, dans laquelle la substance organique cristalline comprend en outre un sucre choisi dans le groupe constitué par l'isomalt, le tréhalose, le raffinose, le lactulose et des mélanges de ceux-ci.

5. Méthode selon la revendication 4, dans laquelle le sucre est le lactulose.

6. Méthode selon l'une quelconque des revendications précédentes, dans laquelle le taux de substance organique cristalline ou de mélanges de plusieurs d'entre elles dans la composition totale est de 0,1 à 5 % en poids.

7. Méthode selon l'une quelconque des revendications précédentes, dans laquelle la composition comprend en outre une silicone.

8. Utilisation de la substance organique cristalline décrite dans la revendication 1 pour lisser les cheveux.

9. Utilisation de la substance organique cristalline décrite dans la revendication 1 pour apporter une résistance à l'humidité aux cheveux.
